(19) Europäisches Patentamt

European Patent Office

Office européen des brevets

(11) **EP 0 878 714 A1**

(12) **EUROPÄISCHE PATENTANMELDUNG**

(43) Veröffentlichungstag:
18.11.1998 Patentblatt 1998/47

(51) Int. Cl.$^6$: **G01N 33/533**, G01N 33/53, G01N 33/58

(21) Anmeldenummer: 98108273.8

(22) Anmeldetag: 06.05.1998

(84) Benannte Vertragsstaaten:
AT BE CH CY DE DK ES FI FR GB GR IE IT LI LU MC NL PT SE
Benannte Erstreckungsstaaten:
AL LT LV MK RO SI

(30) Priorität: 07.05.1997 DE 19719465

(71) Anmelder:
Boehringer Mannheim GmbH
68305 Mannheim-Waldhof (DE)

(72) Erfinder: Weindel, Kurt, Dr.
82407 Wielenbach (DE)

(74) Vertreter:
Weiss, Wolfgang, Dipl.-Chem. Dr. et al
Postfach 86 08 20
81635 München (DE)

(54) **Konservierung von Aequorin-Reagenzien**

(57) Es wird ein Verfahren zur Stabilisierung von Reagenzien für einen Test, bei dem ein Ca-aktivierbares Photoprotein als Komponente verwendet wird, beschrieben, wobei mindestens einem der den Reagenzien eine oder mehrere organische antimikrobiell wirksame Verbindungen zugegeben werden, um für den Test eine Stabilisierung der Photoproteinaktivität zu erreichen, worin nach 30 min Testdauer eine restliche Photoproteinaktivität von mindestens 50 % der ursprünglichen Photoproteinaktivität des Ca-aktivierbaren Photoproteins vorhanden ist.

**Beschreibung**

Die Erfindung betrifft ein Verfahren zur Stabilisierung von Reagenzien für einen Test, bei dem ein Ca-aktivierbares Photoprotein als Komponente verwendet wird. Weiterhin betrifft die Erfindung ein Reagenz sowie einen Testkit zum Nachweis eines Analyten, umfassend ein Ca-aktivierbares Photoprotein.

Lumineszenzmarkierungen haben gegenüber anderen Nachweissystemen wie z. B. radioaktiven Markierungsgruppen den Vorteil einer höheren Sensitivität, was ihre Verwendung in biospezifischen Assays wünschenswert macht. Ein Beispiel für Lumineszenzmarkierungen, die sich für den Einsatz in Nachweisverfahren eignen, sind Photoproteine, z. B. Ca-aktivierbare Photoproteine.

Zu den Ca-aktivierbaren Photoproteinen gehören die Photoproteine der Aequorin-Familie. Aequorin ist ein Photoprotein bestehend aus Apo-Aequorin, der katalytisch aktiven Proteinkomponente, Coelenterazin, der oxidierbaren Luminophorkomponente sowie molekularem, wahrscheinlich als Hydroperoxid an das Protein gebundenen Sauerstoff, dem Oxidationsmittel. Durch Zugabe von $Ca^{2+}$-Ionen (Trigger) kommt es zu einer Lumineszenzreaktion zwischen den einzelnen Komponenten des Reaktionskomplexes. Die Aequorin-Biolumineszenz zeichnet sich durch außergewöhnlich hohe Photonenausbeute ($\geq$ 25 %) und Schnelligkeit (< 5 sec) aus (vgl. Blinks, J. Pharm. Reviews 28 (1976) 1-93).

Da die Lichtreaktion spezifisch durch $Ca^{2+}$-Ionen ausgelöst werden kann, welche zuvor während der Testführung beispielsweise durch Chelatbildner wie EDTA oder EGTA gebunden vorliegen können, ist der chemische Rauschpegel äußerst niedrig. Weiterhin stellt die Hydrolyse im Gegensatz zu anderen lumineszierenden Verbindungen wie beispielsweise Acridinium-Arylesthern oder Phosphophenyl-Adamantyl-Dioxetanen keine Konkurrenzreaktion war, was wiederum zu einer Verringerung des Signalhintergrundes führt. Daraus ergibt sich die Möglichkeit, das emittierte Licht bei hohem Signal zu Rausch-Verhältnis präzise und sensitiv zu messen.

Ein Nachteil von Aequorin und verwandten Photoproteinen ist jedoch, daß sie äußerst empfindlich gegenüber der Anwesenheit von störenden Komponenten, z. B. mikrobiellen Kontaminationen oder chemischen Reagenzien, sind. Die Folgen sind üblicherweise Verlust der katalytischen Aktivität und/oder vorzeitige Entladung ohne Triggerzugabe. Da Aequorin und verwandte Photoproteine fertig ausgebildete Reaktionskomplexe sind, wirken sich Konformationsänderungen und/oder unspezifische chemische Modifikationen besonders nachteilig aus, wobei die Photoproteinaktivität schnell verloren geht.

Ein grundsätzliches Problem stellt deshalb die Konservierung von Reagenzlösungen für Tests dar, bei denen ein Ca-aktivierbares Photoprotein als Komponente verwendet wird. Aufgrund der Empfindlichkeit von Aequorin und verwandten Photoproteinen haben sich viele der typischerweise zur Konservierung diagnostischer Reagenzien verwendeten Biozide als ungeeignet oder mit großen Nachteilen verbunden erwiesen. Insbesondere unspezifisch wirkende Stoffe, wie etwa starke Oxidantien, reaktive Abspalter (z. B. aktives Formaldehyd) und hydrophobe Konformationsmodulatoren (z. B. langkettige oder verzweigte Alkohole und schwach abgeschirmte bzw. substituierte aromatische Ringstrukturen) erwiesen sich als unbrauchbar, da die Photoproteinaktivität der Photoproteine innerhalb kurzer Zeit, zumeist innerhalb weniger Minuten zerstört wurde.

Weiterhin ist es bekannt, Azid als Konservierungsmittel für Aequorin zu verwenden. Die Störung des Photoproteins durch Azid ist zwar relativ gering, Azid selbst hat jedoch nur ein biologisch sehr eingeschränktes Wirkungsspektrum und ist zudem hinsichtlich seiner Handhabung während der Produktion (z.B. Lyophilisation) aufgrund der Explosionsgefahr vor allem von Metallaziden problematisch. Nachteilig sind außerdem hohe Toxizität und Mutagenizität und daraus folgend die Deklarationspflicht als gefährliches Reagenz, und Entsorgungsprobleme. Folglich ist Azid als Konservierungsmittel für kommerzielle Testkits nur bedingt geeignet.

Aufgabe der Erfindung war daher die Bereitstellung eines Verfahrens zur Stabilisierung von Reagenzien für Tests, bei denen ein Ca-aktivierbares Photoprotein als Komponente verwendet wird, wobei das Stabilisierungsmittel die Photoproteinaktivität der Photoproteine nicht wesentlich beeinträchtigen sollte.

Diese Aufgabe wurde gelöst durch ein Verfahren zur Stabilisierung von Reagenzien für einen Test, bei dem ein Ca-aktivierbares Photoprotein als Komponente verwendet wird, wobei man mindestens einem der Reagenzien eine oder mehrere organische antimikrobiell wirksame Verbindungen zugibt, um für den Test eine Stabilisierung der Photoproteinaktivität zu erreichen, worin nach 30 min Testdauer eine restliche Photoproteinaktivität von mindestens 50 % der ursprünglichen Photoproteinaktivität des Ca-aktivierbaren Photoproteins vorhanden ist. Überraschenderweise wurde festgestellt, daß die notwendige Konservierung von Ca-aktivierbaren Photoproteinen unter zumindest weitgehendem Erhalt der Photoproteinaktivität möglich ist, wenn man bestimmte organische antimikrobiell wirkende Verbindungen einsetzt. Unter organischen antimikrobiell wirksamen Verbindungen werden hierin antimikrobiell wirksame Verbindungen verstanden, welche mindestens eine C-C-Bindung enthalten.

Der Anteil der Photoproteinaktivität oder Lumineszenzaktivität nach einer vorgegebenen Inkubationszeitdauer bzw. Testdauer von der ursprünglichen Photoproteinaktivität oder Ausgangsaktivität wird im weiteren als Photorecovery bezeichnet. Bevorzugt wird durch die erfindungsgemäße Zugabe von organischen antimikrobiell wirksamen Verbindungen eine Photorecovery nach 60 min von mindestens 50 %, mehr bevorzugt mindestens 60 % und besonders bevorzugt mindestens 70 % erhalten. Am meisten ist es bevorzugt, antimikrobiell wirksame Verbindungen zuzugeben, mit denen nach 60 min Testdauer eine Photorecovery von mindestens 80 % oder mindestens 90 % erhalten wird.

Geeignete antimikrobiell wirksame Verbindungen sind einerseits beispielsweise moderat reaktive Alkylierungreagenzien, wie etwa 2-Chloracetamid und andererseits antimikrobiell wirksame Prozessinhibitoren.

Bevorzugt werden als organische antimikrobiell wirksame Verbindungen Prozess-Inhibitoren eingesetzt. Unter Prozess-Inhibitoren werden antimikrobiell wirksame Verbindungen verstanden, die bestimmte biochemische oder molekularbiologische Prozesse spezifisch hemmen. Geeignete antimikrobiell wirksame Verbindungen können aus den β-Lactam-Antibiotika, den Aminoglykosid-Antibiotika, den Xenobiotika, den aromatischen Antibiotika, den Ansamycinen, den Azol- oder Imidazolderivaten, den Tetracyclinen oder/und den Sulfonamidantibiotika ausgewählt werden. Beispiele für erfindungsgemäß geeignete Prozess-Inhibitoren sind Sulfamethaxol, ein Sulfonamid-Antibiotikum, welches ein Anti-Metabolit für die bakterielle Folat-Synthese ist; Trimethoprim, welches ein Dihydrofolat-Reduktase-Inhibitor ist und somit den C1-Metabolismus, also unter anderem die Purin-Synthese hemmt; Ketokonazol, welches ein Inhibitor der Steroidbiosynthese bei Pilzen, insbesondere der Umwandlung von Lanosterol in Ergosterol ist, und damit eine Schädigung der Zellmembran bewirkt; Tetracyclin, welches ein Translations-Inhibitor ist und mit der Elongation über die Blockierung der Anlagerung von Aminoacyl-tRNA interferiert; Chloramphenicol, ein aromatisches Antibiotikum, welches ein Translations-Inhibitor ist und mit der Elongation über die Hemmung der Peptidyl-Transferase an den 70S Ribosomen von Bakterien interferiert; Gentamycin und Tobramycin, welche Aminoglykosid-Antibiotika darstellen und Translations-Inhibitoren sind, welche mit der Initiation über die Blockierung der Bindung von N-Formylmethionyl-tRNA am 30S Ribosomen von Bakterien sowie der Anlagerung von Aminoacyl-tRNA interferieren; Rifampicin, ein Ansamycin, welches ein Transkriptions-Inhibitor ist und die bakterielle DNA-abhängige RNA-Polymerase durch Anlagerung an die β-Untereinheit hemmt; Penicilline oder Ce-phalosporine, welche mit der Bildung der bakteriellen Zellwand durch Hemmung der D-Alanyl-Transpeptidase interferieren sowie Antimycin, welches ein Atmungskettenblocker (Zellgift) ist.

Entscheidend für die Eignung von organischen antimikrobiell wirksamen Verbindungen im erfindungsgemäßen Verfahren ist, daß sie mit einem Ca-aktivierbaren Photoprotein kompatibel sind, d.h. eine Stabilisierung der Photoproteinaktivität ermöglichen. Zur Identifizierung geeigneter Verbindungen ist die Beachtung bestimmter struktureller Kriterien bevorzugt. So wurde festgestellt, daß beispielsweise bei antimikrobiell wirksamen Verbindungen, die einen schwach abgeschirmten aromatischen Ring, z. B. einen Benzolring aufweisen, ein deutlicher Verlust an Photoproteinaktivität auftreten kann.

Als ungeeignet haben sich Biozide erwiesen, welche die strukturellen Voraussetzungen für unspezifische hydrophobe Wechselwirkungen oder Ionenpaarbindungen, und somit Konformationsmodulationen erfüllen. Solche ungeeigneten Strukturmerkmale sind beispielsweise ein schwach abgeschirmter aromatischer oder quasi-aromatischer Ring oder langkettige oder verzweigte Alkohole. Unsubstituierte Alkan-, Alken- oder Alkinkettensegmente sollten vorzugsweise nicht mehr als 4 C-Atome in Reihe aufweisen. Ebenfalls als ungeeignet haben sich Biozide mit quarternären Ammonium- oder Phosphoniumgruppen erwiesen. Bei erfindungsgemäß geeigneten Bioziden ist ein gegebenenfalls vorhandener aromatischer Ring abgeschirmt, z. B. enthält er ≥ 2 Substituenten, welche bevorzugt polar sind. Diese Substituenten weisen dabei jeweils bevorzugt mindestens 3 Atome und besonders bevorzugt mehr als 5 Atome auf. Es ist aber auch möglich, aromatische Ringe durch nur einen, raumfüllenden Substituenten (bevorzugt ≥ 20 Atome) abzuschirmen, wobei jedoch Teile dieses Substituenten nicht-aromatisch sein müssen.

Aromatische Ringe können auch als Bestandteile von polyzyklischen Systemen durch sie umgebende, nichtaromatische Bestandteile des Ringsystems abgeschirmt werden.

Bevorzugt wird eine antimikrobiell wirksame Verbindung zugegeben, die in einem Lösungsmittel löslich ist, welches mit dem Testpuffer mischbar ist, wie beispielsweise DMSO, DMF, Ethanol, Methanol, Ether oder/und Aceton. Diese Lösungsmittel bilden zusammen mit einem wäßrigen Testpuffer ein homogenes Gemisch. Der Wirkstoff wird dabei bevorzugt in einer Konzentration von 0,001 % (w/v) bis 5 % (w/v) besonders bevorzugt von 0,01 % (w/v) bis 1 % (w/v) und am meisten bevorzugt in einer Konzentration von 0,05 % bis 0,5 % (w/v) jeweils bezogen auf das Volumen der gesamten Testlösung eingesetzt.

Vorteilhaft wird eine antimikrobiell wirksame Verbindung zugegeben, welche kein Arzneimittel ist, um die Gefahr einer Bildung von Resistenzen gering zu halten. Ein Beispiel für eine solche Substanz ist Antimycin A.

Das Ca-aktivierbare Photoprotein wird bevorzugt ausgewählt aus der Gruppe bestehend aus Aequorin, Obelin, Clytin, Mitrocomin, Berovin, Mnemiopsin und Thalassicolin. Besonders bevorzugt wird Aequorin, insbesondere rekombinantes Aequorin verwendet.

Diesen Photoproteinen ist gemeinsam, daß es sich um lumineszenzfähige Proteine handelt, die einen Reaktionskomplex aus Protein (Apo-Photoprotein), einem organisch-chemischen Substrat, welches fest, aber nicht kovalent gebunden ist und den Photoemitter darstellt und ebenfalls fest fixiertem molekularem Sauerstoff, der erforderlich für die Inizierung der Lumineszenzreaktion (Oxidant) ist, darstellen. Die Lumineszenzreaktion wird durch Bindung von $Ca^{2+}$-Ionen an das Apo-Photoprotein ausgelöst und ist vom Sauerstoff aus der Umgebung unabhängig.

Besonders bevorzugt weisen Ca-aktivierbare Photoproteine folgende gemeinsame Merkmale auf:

a) physiko-chemische Aspekte:

- fertig ausgebildeter Reaktionskomplex aus einem proteinösen Katalysator (=Apo-Photoprotein), einem organischchemischen Substrat, welches fest, aber nicht kovalent gebunden ist und den eigentlichen Emitter darstellt (=Luminophor), und ebenfalls fest fixiertem molekularem Sauerstoff (vermutlich kovalent Protein-gebunden als Hydroperoxid), der erforderlich ist für die Initiierung der Lumineszenzreaktion (=Oxidans)
- Unterscheidung von Enzymsystemen durch Gebundensein aller für die Lumineszenzreaktion notwendigen Komponenten
- relative Molmasse im Bereich von ca. 22000 Dalton
- "Coelenterazin" ([2-(p-Hydroxybenzyl)-6-(p-hydroxyphenyl)-8-benzyl-7-hydroimidazopyrazin-3-on] als natürli-ches Luminophor
- Emissionspeakwellenlänge ($\lambda$max) im Blaubereich (ca. 470 nm)
- Auslösung der Lumineszenzreaktion durch Bindung von 2-3 $Ca^{2+}$-Ionen an das Apo-Photoprotein
- Vorhandensein von $Ca^{2+}$-Bindedomänen im Apo-Photoprotein in Form von EF-Hand-(=Helix-Schleife-Helix-)strukturen
- Unabhängigkeit der Lunineszenzreaktion von Sauerstoff aus der Umgebung, d. h. das komplette Photoprotein luminesziert in Gegenwart wie auch in Abwesenheit von $O_2$ in der umgebenden Atmosphäre
- Photoprotein-Reaktion verläuft in Form einer Blitzkinetik, d. h. konzentrierte Lichtemission als Folge von $Ca^{2+}$-Ionen-Zugabe (siehe hierzu Blinks J. R., et al., Pharmacological Reviews 28/1, 1-93, 1976)

b) molekularbiologische Aspekte

- funktionstaugliche Apo-Photoproteine mit einer Länge von im allgemeinen 189-196 Aminosäuren
- hohe Übereinstimmung der Nucleobasen- und Aminosäuresequenzen ($\geq$60 % Homologie) der einzelnen Ver-treter der Familie der $Ca^{2+}$ aktivierbaren Photoproteine.

Die Ca-aktivierbaren Photoproteine können als native Proteine, die aus ihren Ursprungsorganismen isoliert wurden, oder als rekombinante Proteine, z. B. aus E.coli eingesetzt werden. Die Verwendung rekombinanter Photoproteine ist bevorzugt. Aequorin stammt beispielsweise aus dem Organismus Aequorea victoria. Die Klonierung und die rekombinante Herstellung von Aequorin in E.coli ist von Prasher et al., Biochem. Biophys. Res. Comm. 126/3 (1995), 1259-1268, Prasher et al., Meth. Enzymol 133 (1986), 288-299, Prasher et al., Biochemistry 26 (1987), 1326-1332, Cormier et al., Photochemistry and Photobiology 49/4 (1989), 509-512 und Stults et al., Biochemistry 31 (1992), 1433-1442 beschrieben. Die Klonierung, Expression und Sequenz des aus dem Organismus Obelia longissima stammenden Photoproteins Obelin sind bei Illarionov et al., J. Biolumineszenz und Chemoluminieszenz 8 (1993), VII. International Symposium-Abstracts 88 und Gene 153 (1995), 273-274 beschrieben. Klonierung, Expression und Sequenzanalyse der Photoproteine Clytin und Mitrocomin sind bei Inouye und Tsuji (FEBS 315 (1993), 343-346) und Fagen et al. (FEBS 333 (1993), 301-305) beschrieben. Weitere Mitglieder der Aequorin-Familie sind die Photoproteine Berovin und Mnemiopsin (Ward und Seliger, Biochemistry 13 (1974), 1491-1499 und 1500-1510) und Thalassicolin (Campbell et al., Application of the photoprotein obelin to the measurement of $Ca^{2+}$ in cells. In: Bioluminescence and Chemiluminescence, Hrsg. DeLuca M. A. & McElroy, W. D., Seiten 601-607 (1981), Academic Press, New York). Auf die in den oben genannten Literaturstellen enthaltende Offenbarung zur Gewinnung von Ca-aktivierbaren Photoproteinen wird hiermit Bezug genommen.

Üblicherweise liegt das Ca-aktivierbare Photoprotein in Form eines Konjugats mit einem Bindepartner vor. Das Konjugat kann dabei beispielsweise aus einem Hapten und einem Calcium-aktivierbaren Photoprotein gebildet werden. Es ist auch möglich, als Bindepartner ein Polypeptid, ein Immunglobulin oder ein Immunglobulinfragment, eine Nucleinsäure oder ein Nucleinsäureanalogon zu verwenden. Solche Photoproteinkonjugate sind beispielsweise in der US-A-5,486,455, in der DE 44 38 660 sowie in der DE 196 52 576 beschrieben.

Ein weiterer Gegenstand der Erfindung betrifft die Verwendung einer organischen antimikrobiell wirksamen Verbindung insbesondere eines Prozess-Inhibitors, welche mit Ca-aktivierbaren Photoproteinen kompatibel ist, zur Stabilisierung von Reagenzien für Test, bei denen ein Ca-aktivierbares Photoprotein als Komponente verwendet wird.

Weiterhin umfaßt die Erfindung ein Reagenz zum Nachweis eines Analyten, z.B. in einem Immunoassay oder in einem Nucleinsäurehybridisierungsassay, welches ein Ca-aktivierbares Photoprotein und eine damit kompatible organische antimikrobiell wirksame Verbindung enthält.

Schließlich betrifft die vorliegende Erfindung einen Testkit zum Nachweis eines Analyten, z.B. in einem Immunoassay oder in einem Nucleinsäurehybridisierungsassay, umfassend Reagenzien für einen Test, bei dem ein Ca-aktivierbares Photoprotein als Komponente verwendet wird, wobei in dem Testkit neben einem Ca-aktivierbaren Photoprotein eine damit kompatible, organische antimikrobiell wirksame Verbindung enthalten ist. Bevorzugt liegen das Ca-aktivierbare Photoprotein und die damit kompatible organische antimikrobiell wirksame Verbindung als separate Komponenten vor. Beispielsweise kann das Ca-aktivierbare Photoprotein in lyophilisierter Form in dem Testkit enthalten sein und der zur Aufnahme des lyophilisierten Photoproteins bestimmte Puffer enthält die kompatible organische antimikrobiell wirksame Verbindung. Es ist aber auch möglich, daß das Ca-aktivierbare Photoprotein bereits als mit der kompatiblen organischen antimikrobiellen wirksamen Verbindung stabilisiertes Reagenz vorliegt.

Weiterhin können auch Pufferlösungen oder/und weitere Reagenzlösungen die organische antimikrobiell wirksame Verbindung und insbesondere einen Prozess-Inhibitor enthalten.

Ein weiterer Gegenstand der Erfindung ist ein Verfahren zum Nachweis eines Analyten durch einen Test, bei dem ein Ca-aktivierbares Photoprotein als Komponente verwendet wird, wobei man den Test in Gegenwart eines Stabilisierungsreagenz für das Ca-aktivierbare Photoprotein durchführt, umfassend eine oder mehrere organische antimikrobiell wirksame Verbindungen, worin nach 30 min Testdauer eine restliche Photoproteinaktivität mindestens 50% der ursprünglischen Photoproteinaktivität des Ca-aktivierbaren Photoproteins vorhanden ist.

Die Erfindung wird durch die folgenden Beispiele weiter erläutert.

**Beispiel 1 Methodik zum Nachweis des Einflusses von Wirkstoffen auf Aequorin**

Es wurde der Einfluß verschiedener Wirkstoffe auf unmodifiziertes, rekombinantes Aequorin (Fa. SeaLite Siences, Inc. Atlanta, USA) und auf Antikörper- bzw. Oligonukleotid-Aequorin-Konjugate getestet. Dazu wurden die Wirkstoffe in einem jeweils geeigneten Lösungsmittel (DMSO, EtOH, MeOH, NaOH-Lösung) gelöst, welches parallel als Lösungsmittelkontrolle zur Korrektur der Wirkstoff-Effekte mitgeführt wurde. Üblicherweise wurden die Wirkstoffe in einer Konzentration von 0,1 % (w/v), bezogen auf die gesamte Testlösung getestet, während die Stammlösungen auf 2 % (w/v) eingestellt waren. Aequorin bzw. die Aequorin-Konjugate wurden dann mit dem jeweiligen Wirkstoff bzw. mit dem Lösungsmittel ohne Wirkstoff inkubiert. Zu verschiedenen Zeitpunkten wurde die Photoproteinaktivität gemessen und die Restaktivität in % unter Berücksichtigung der reinen Lösungsmitteleffekte nach folgender Gleichung berechnet:

$$[(t_i/t_0) \text{ Wirkstoff: } (t_i/t_0) \text{ Solvens}] \times 100$$

Als Testträger wurden miniaturisierte Rundboden-Reaktionsgefäße (Mini-Dispos; Boehringer Mannheim) und ein für diese Testträger angepaßtes Spezialluminometer bzw. kommerzielle weiße Mikrotiterplatten (FluoroNunc™ MaxiSorp, Fa. Nunc) und ein Plattenluminometer (ML3000™, Fa. Dynatech Laboratories) verwendet. Die Auswertung der Messungen erfolgte über die Integration der Signale über ein Intervall von 0 bis 2 Sekunden nach Triggerzugabe beim Spezialluminometer bzw. 0,1 Sekunden vor und 1 Sekunde nach dem Peak beim Platten-Luminometer.

**Beispiel 2 Ergebnisse von Versuchen der Konservierung von Aequorin mit ungeeigneten Bioziden (Vergleichsbeispiel)**

In der in Beispiel 1 beschriebenen Weise wurden typischerweise zur Konservierung diagnostischer Reagenzien verwendete unspezifisch reaktive Biozide untersucht. Geprüft wurden die Biozide Bioban, Myacid, Oxipyrion, Kathon, o-Phenylphenol/alkalisch, o-Phenylphenol/alkoholisch und Methylisothiazolon. Quecksilberhaltige Konservierungsmittel wurden von vorneherein ausgesondert, da für die Photoproteinaktivität von Aequorin und verwandten Verbindungen SH-Gruppen essentiell sind und Schwermetalle wie Hg daher grundsätzlich unverträglich sind. Die Ergebnisse der Untersuchungen sind als % Restaktivität als Funktion der Kontaktzeit zwischen der jeweiligen Biozid-Lösung und dem Aequorin-Präparat in Tabelle 1 zusammengefaßt, dessen Photoproteinaktivität vor der Zugabe der Biozide jeweils 100 betrug.

Wie aus Tabelle 1 zu sehen ist, wird die Photoproteinaktivität von Aequorin durch die zur Konservierung verwendeten Biozide innerhalb kürzester Zeit (Kontaktzeit < 1 min) wesentlich verringert. Nach einer Kontaktzeit von 30 min ist in den meisten Fällen keine Photoproteinaktivität von Aequorin mehr nachweisbar. So bewirkt z. B. o-Phenylphenol, welches zwei nicht abgeschirmte aromatische Ringe enthält die als hydrophobe Konformationsmodulatoren wirken, eine äußerst schnelle Zerstörung der Photoproteinaktivität von Aequorin (Tabelle 1a). In gleicher Weise bewirkten Lösungen, die mit jeweils 0,1 % Methylisothiazolon und Oxypyrion konserviert waren, bei Kontakt mit MAK⟨TSH⟩(IgG)-Aequorin-Konjugat eine rasche Zerstörung der Photoaktivität. Bei Kathon cg ist sehr gut die Konzentrationsabhängigkeit der Zerstörung von Aequorin durch dieses Biozid ersichtlich (Tab. 1b).

Tabelle 1: Einfluß typischer, für Diagnostica verwendeter Biozide auf die Aequorin-Photoproteinaktivität

1a:

Stammlösungen, 2%-ig an Biozid, mit Tris-Puffer pH 7,4/2 mM
EDTA 1:10 verdünnt, damit wurde eine Aequorin-Stammlösung um
den Faktor $1 : 10^6$ verdünnt.

| | Testlösungen | | | | |
|---|---|---|---|---|---|
| Kon-takt-zeit | Myazid | o-Phenylphenol/ alkoholisch | o-Phenylphenol/ alkalisch | Bioban | Bioban 0,5% |
| < 1 min | 18,33 | 3,54 | 0,03 | 49,78 | 14,96 |
| 30 min | n.b. | n.b. | n.b. | | |
| 60 min | | | | 28,01 | 2,60 |
| 75 min | n.b. | n.b. | n.b. | | |

n.b. = nicht bestimmt, da die Aktivität bereits dramatisch
reduziert war

<u>1b:</u>

Rinderserumalbumin-Matrix mit 0,1 % Kathon cg konserviert; in jeweils unterschiedlichen Anteilen einer 1 : $10^6$ verdünnten Aequorin-Stammlösung zugesetzt.

A:   1 Teil Biozidlösung + 5 Teile Aequorinlösung
B:   1 Teil Biozidlösung + 1 Teil Aequorinlösung
C:   reine Aequorinlösung

| | Testlösungen | | |
|---|---|---|---|
| Kontaktzeit | A | B | C |
| < 1 min | 81,79 | 47,86 | 100,00 |
| 60 min | 35,05 | 2,00 | - |
| 105 min | 22,78 | 0,71 | 94,55 |

**Beispiel 3 Ergebnisse mit strukturell geeigneten Bioziden**

In der in Beispiel 1 beschriebenen Weise wurden weitere Biozide untersucht, die anhand ihrer Reaktivität und ihrer strukturellen Voraussetzungen für die Stabilisierung von Aequorin bedingt geeignet sind. Geprüft wurden die Biozide Germall 115 (Wirkstoff: Imidazolidinylharnstoff) und 2-Chloracetamid. Die Ergebnisse der Untersuchungen sind als Prozent Restaktivität als Funktion der Kontaktzeit zwischen der jeweiligen Biozid-Lösung und dem Aequorin-Präparat in Tabelle 2 zusammengefaßt.

Tabelle 2

| Einfluß erfindungsgemäßer Biozide auf die Aequorin-Photoproteinaktivität | | |
|---|---|---|
| Kontaktzeit | Germall 115 | 2-Chloracetamid |
| < 1 min | 93,64 | 88,05 |
| 30 min | 76,40 | 89,19 |
| 75 min | 70,23 | 88,12 |

Wie aus den Ergebnissen zu ersehen ist, kann mit erfindungsgemäß geeigneten Bioziden eine beträchtliche Prozent Restaktivität von Aequorin selbst nach einer Kontaktzeit von 75 min erhalten werden. Im Gegensatz dazu wird die Photoaktivität von Aequorin durch strukturell ungeeignete oder zu reaktive Biozide bereits nach kurzer Einwirkzeit wesentlich zerstört. 2-Chloracetamid ist als moderat reaktives Alkylierungsreagenz einzustufen, welches Aequorin besonders gut verträglich ist.

**Beispiel 4 Ergebnisse mit spezifischen Prozessinhibitoren**

In der in Beispiel 1 beschriebenen Weise wurden spezifische Prozessinhibitoren untersucht. Geprüft wurden Gentamicin und Tobramycin. Die Ergebnisse dieser Untersuchungen sind als Prozent Restaktivität als Funktion der Kontaktzeit zwischen der jeweiligen Biozid-Lösung und dem Aequorin-Präparat in Tabelle 3 zusammengefaßt.

Tabelle 3

| Einfluß erfindungsgemäßer, spezifischer Prozeßinhibitoren auf die Aequorin-Photoproteinaktivität Einfluß von Aminoglycosid-Antibiotica (Translations-Inhibitoren) auf unmodifiziertes Aequorin; als Ausgangswert wurde eine 1:$10^6$ in Tris pH 7.4/2mM EDTA verdünnte Aequorinlösung verwendet. | | |
|---|---|---|
| Testlösungen: | | |
| **Kontaktzeit** | **Gentamycin 0,10 % (w/v)** | **Tobramycin 0,10 % (w/v)** |
| < 1 min | 102,53 | 105,86 |
| 30 min | 104,83 | 91,54 |
| 75 min | 108,20 | 98,08 |
| ca. 24 Std. | 105,02 | 95,06 |

Wie aus Tabelle 3 zu sehen ist, kann mit strukturell geeigneten Prozeßinhibitoren eine Konservierung ohne eine negative Beeinflussung der Photoproteinaktivität von Aequorin erhalten werden. Die Photoproteinaktivität von Aequorin war selbst nach 24 Stunden Einwirkzeit der Biocide im wesentlichen unverändert.

**Beispiel 5 Einfluß einer Mischung von Gentamycin und 2-Chloracetamid auf ein Aequorin-Konjugat.**

Es wurde eine Mischung von jeweils 0,1 % (w/v) Gentamicin und 2-Chloracetamid hergestellt. Der Einfluß dieser Mischung auf das MAK⟨TSH⟩(IgG)-Aequorin-Konjugat wurde untersucht. Die Ergebnisse der Untersuchung als Prozentrestaktivität als Funktion der Kontaktzeit zwischen der Biozid-Lösung und dem Aeqorin-Konjugat sind in Tabelle 4 aufgeführt.

Tabelle 4

| Einfluß einer Mischung von Gentamycin + 2-Chloracetamid (jeweils @ 0,1 %) auf ein MAK⟨TSH⟩(IgG)-Aequorin-Konjugat | |
|---|---|
| **Kontaktzeit** | **Gentamycin + 2-Chloracetamid 0,1 % + 0,1 %** |
| < 1 min | 110,00 |
| 15 min | 101,30 |
| 30 min | 98,30 |
| 45 min | 98,80 |
| 60 min | 97,10 |

Wie aus Tabelle 4 ersichtlich ist, kann eine Konservierung von Testlösungen unter Beibehaltung der Photoproteinaktivität von Aequorin auch durch eine Kombination von geeigneten Biociden erreicht werden.

**Beispiel 6**

Schließlich wurde die Wirkung verschiedener erfindungsgemäßer Biozide auf die Aequorin-Photoproteinaktivität bei verschiedenen Temperaturen, nämlich 25 °C und 37 °C untersucht. Die Ergebnisse, denen 8-fach Bestimmungen zugrundeliegen, sind in den Tabellen 5 und 6 dargestellt.

**Tabelle 5**  Retention der Aequorin-Photoproteinaktivität in Gegenwart verschiedener Biocide @ 0.1% (w/v) bei 37°C, welche als biochemische Prozeß-Inhibitoren wirken

| anti-mikrobielle Substanz | Interferenzpunkt | Solvens $f^a$ | % recovery als f · (t), korrigiert für Solvens-Effekte | | | | |
|---|---|---|---|---|---|---|---|
| | | | Start | + 15 min | + 30 min | + 60 min | + 120 min |
| Sulfamethoxazol | Anti-Metabolit für Folat-synthese | DMSO | 100 | 114 | 120 | 97 | 91 |
| Tetracyclin | Translationsinhibitor (blockt Elongation) | DMSO | 100 | 86 | 84 | 53 | 23 |
| p-Aminosalicylsäure* | Anti-Metabolit für Folat-synthese | DMSO | 100 | 3.8 | 2.9 | 1.4 | 0.3 |
| Trimethoprim | Inhibitor der Dihydrofolat-reductase (C1-Metabol.) | DMSO | 100 | 83 | 100 | 73 | 76 |
| Chloramphenicol | Translationsinhibitor (blockt Elongation) | DMSO | 100 | 97 | 103 | 96 | 90 |
| Phenoxymethyl-penicillin | inhibiert bakterielle D-Alanin-transpeptidase | $H_2O$ pH 10.9 | 100 | 103 | 97 | 99 | 103 |
| Rifampicin | inhibiert DNA-abhän-gige RNA-Polymerase | MeOH | 100 | 103 | 99 | 99 | 96 |
| Gentamicin | Translationsinhibitor (blockt Initiation) | $H_2O$ | 100 | 93 | 101 | 92 | 97 |
| 2-Chloracetamid | moderates Alkylierungs-mittel | DMSO | 100 | 94 | 101 | 93 | 97 |

$f^a$ = Wirkstoff-Stammlösung @ 2% (w/v)

EP 0 878 714 A1

EP 0 878 714 A1

Wie aus den Tabellen 5 und 6 ersichtlich ist, kann mit erfindungsgemäß geeigneten antimikrobiell wirksamen Verbindungen eine Stabilisierung der Photoproteinaktivität von Aequorin auch bei erhöhten Temperaturen über einen langen Zeitraum erzielt werden. Die bei der Verwendung von $CHCl_3$ als Lösungsmittel auftretenden Schwankungen sind

Tabelle 6    Retention der Aequorin-Photoproteinaktivität in Gegenwart verschiedener Biocide @ 0.1% (w/v) bei 25°C, welche als biochemische Prozeß-Inhibitoren wirken

| anti-mikrobielle Substanz | Interferenzpunkt | Solvens $/^a$ | % recovery als $f \cdot t_i$, korrigiert für Solvens-Effekte | | | | | |
|---|---|---|---|---|---|---|---|---|
| | | | Start | + 15 min | + 30 min | + 60 min | + 90 min | + 120 min |
| DNA-Aequorin pur in Testpuffer | | | 100.0 | 96.9 | 95.4 | 98.0 | 97.9 | 91.9 |
| $NaHCO_3$-Pufferleerwert | | | 100.0 | 98.9 | 93.3 | 90.7 | 83.9 | 90.9 |
| DMSO-Leerwert | | | 100.0 | 95.1 | 93.9 | 99.0 | 95.1 | 91.6 |
| $CHCl_3$-Leerwert $/^b$ | | | 100.0 | 97.2 | 90.2 | 44.5 | 116.7 | 142.8 |
| Ketoconazol | blockiert Ergosterol-synthese | $CHCl_3$ $/^b$ | 100.0 | 74.6 | 77.1 | 155.3 | 67.1 | 56.4 |
| Clotrimazol $/^c$ | Anti-Metabolit für Folat-synthese | DMSO | 100.0 | 100.9 | 97.2 | 96.3 | 95.0 | 95.6 |
| Antimycin A | Atmungskettenblocker | $CHCl_3$ $/^b$ | 100.0 | 171.1 | 113.4 | 265.4 | 115.5 | 98.9 |
| Benzylpenicillin | inhibiert bakterielle D-Alanin-transpeptidase | 0.1 M $NaHCO_3$ pH 8.0 $/^d$ | 100.0 | 93.6 | 98.2 | 106.0 | 105.3 | 97.5 |
| Phenoxymethyl-Pen. | inhibiert bakterielle D-Alanin-transpeptidase | 0.1 M $NaHCO_3$ pH 8.0 $/^d$ | 100.0 | 98.2 | 98.4 | 101.9 | 110.9 | 98.0 |
| Oxacillin | inhibiert bakterielle D-Alanin-transpeptidase | 0.1 M $NaHCO_3$ pH 8.0 $/^d$ | 100.0 | 99.7 | 106.8 | 108.6 | 107.9 | 103.0 |
| Mezlocillin | inhibiert bakterielle D-Alanin-transpeptidase | 0.1 M $NaHCO_3$ pH 8.0 $/^d$ | 100.0 | 101.1 | 105.6 | 108.2 | 117.5 | 102.2 |
| Sulfamethoxazol | Anti-Metabolit für Folat-synthese | DMSO | 100.0 | 101.8 | 102.6 | 95.8 | 99.5 | 100.8 |

$/^a$ = Wirkstoff-Stammlösung @ 2% (w/v)

$/^b$ = ergibt mit Testpuffer nur eine m.o.w. stabile Emulsion, keine homogene Lösung => Schwankungen, und Wirkstoffaktivität evtl. <0.1% (w/v)

$/^c$ = Sulfamethoxazol + Trimethoprim (jeweils in DMSO gelöst) im Verhältnis 5 : 1 gemischt

$/^d$ = um sicherzustellen, daß die intakte Penicillin-Ringstruktur zur Wirkung kommt

wahrscheinlich darauf zurückzuführen, daß CHCl$_3$ mit dem verwendeten Testpuffer nur eine Emulsion, nicht aber eine homogene Lösung ergibt, so daß hier Schwankungen in der Verteilung der einzelnen Komponenten auftreten können. Trotz der Schwankungen ist den Meßwerten jedoch zu entnehmen, daß die Zugabe der antimikrobiell wirksamen Substanzen Ketokonazol und Ahtimycin A die Photoproteinaktivität von Aequorin nicht wesentlich zerstört.

Im Hinblick auf die strukturellen Merkmale der gestesteten Biozide insbesondere auf die Abschirmung aromatischer Ringe durch unterschiedliche Substituenten zeigt sich in den Tabellen 5 und 6 eine plausible Abstufung. Die Photoproteinrestaktivität sinkt beispielsweise in der Reihenfolge Chloramphenicol > Sulfamethoxazol >>> P-Aminosalicylsäure. Damit korrespondiert eine Abnahme der Raumerfüllung durch die Substituenten am aromatischen Ring bei den jeweiligen Verbindungen.

Entsprechend nachvollziehbar ist auch die Höhe der Photoproteinrestaktivität abhängig vom Grad der Randstellung aromatisch-hydrophober Strukturelemente in komplexeren Molekülstrukturen. In der Reihenfolge Rifampicin, Antimycin, Ketoconazol, Tetracyclin fällt die Photoproteinrestaktivität und steigt die Exponiertheit aromatisch-hydrophober Ringe. So enthält Rifampicin einen Naphtholkern, der mit drei voluminösen Resten substituiert und in einen komplexen, nicht-aromatischen Heterozyklus integriert ist. Antimycin enthält einen Benzolring, der durch Substituion an drei Positionen abgeschirmt ist. In Ketoconazol ist ein Imidazol-Benzolring vorhanden und gut zugänglich. Bei Tetracyclin ist der Phenolring an einer Flanke abgeschirmt. Dieselbe Tendenz zeigen auch die getesteten Penicillinderivate, die alle das β-Lactamgerüst als voluminöses, abschirmendes Strukturelement tragen. Die in α-Stellung zum Benzylrest in der Seitenkette schwächer abgeschirmten Benzyl- und Phenoxymethylpenicilline zeigen nach 120 min eine etwas geringere Photoproteinrestaktivität als die stark abgeschirmten Substanzen Mezlocillin und Oxacillin.

## Patentansprüche

1. Verfahren zur Stabilisierung von Reagenzien für einen Test, bei dem ein Ca-aktivierbares Photoprotein als Komponente verwendet wird,
   **dadurch gekennzeichnet,**
   daß mindestens einem der Reagenzien eine oder mehrere organische antimikrobiell wirksame Verbindungen zugegeben werden, um für den Test eine Stabilisierung der Photoproteinaktivität zu erreichen, worin nach 30 min Testdauer eine restliche Photoproteinaktivität von mindestens 50 % der ursprünglichen Photoproteinaktivität des Ca-aktivierbaren Photoproteins vorhanden ist.

2. Verfahren nach Anspruch 1,
   **dadurch gekennzeichnet,**
   daß nach 60 min Testdauer eine restliche Photoproteinaktivität von mindestens 50 % der ursprünglichen Photoproteinaktivität des Ca-aktivierbaren Photoproteins vorhanden ist.

3. Verfahren nach einem der vorhergehenden Ansprüche,
   **dadurch gekennzeichnet,**
   daß als antimikrobiell wirksame Verbindungen Prozess-Inhibitoren eingesetzt werden.

4. Verfahren nach einem der vorhergehenden Ansprüche,
   **dadurch gekennzeichnet,**
   daß die antimikrobiell wirksamen Verbindungen ausgewählt werden aus β-Lactam-Antibiotika, Aminoglykosid-Antibiotika, Xenobiotica, aromatischen Antibiotika, Ansamycinen, Azol- oder Imidazolderivaten, Tetracyclinen oder/und Sulfonamid-Antibiotika.

5. Verfahren nach einem der vorhergehenden Ansprüche,
   **dadurch gekennzeichnet,**
   daß antimikrobiell wirksame Verbindungen zugegeben werden, die in einem Lösungsmittel löslich sind, welches mit dem Testpuffer mischbar ist.

6. Verfahren nach Anspruch 5,
   **dadurch gekennzeichnet,**
   daß antimikrobiell wirksame Verbindungen zugegeben werden, welche in DMSO, DMF, Ethanol, Methanol, Ether oder/und Aceton löslich sind.

7. Verfahren nach einem der vorhergehenden Ansprüche,
   **dadurch gekennzeichnet,**
   daß antimikrobiell wirksame Verbindungen zugegeben werden, welche keine Arzneimittel sind.

8. Verfahren nach einem der vorhergehenden Ansprüche,
   **dadurch gekennzeichnet,**
   daß das Ca-aktivierbare Photoprotein ausgewählt wird aus der Gruppe bestehend aus Aequorin, Obelin, Clytin, Mitrocomin, Berovin, Mnemiopsin und Thalassicolin.

9. Verfahren nach Anspruch 8,
   **dadurch gekennzeichnet,**
   daß als Ca-aktivierbares Photoprotein Aequorin, insbesondere rekombinantes Aequorin verwendet wird.

10. Verwendung von organischen antimikrobiell wirksamen Verbindungen, die mit Ca-aktivierbaren Photoproteinen kompatibel sind, zur Stabilisierung von Reagenzien für Tests, bei denen ein Ca-aktivierbares Photoprotein als Komponente verwendet wird.

11. Reagenz zum Nachweis eines Analyten,
    **dadurch gekennzeichnet,**
    daß es ein Ca-aktivierbares Photoprotein und eine damit kompatible organische antimikrobiell wirksame Verbindung enthält.

12. Testkit zum Nachweis eines Analyten umfassend Reagenzien für einen Test, bei dem Ca-aktivierbares Photoprotein als Komponente verwendet wird,
    **dadurch gekennzeichnet,**
    daß neben dem Ca-aktivierbaren Photoprotein eine damit kompatible organische antimikrobiell wirksame Verbindung enthalten ist.

13. Testkit nach Anspruch 12,
    **dadurch gegekennezeichnet,**
    daß das Ca-aktivierbare Photoprotein und die damit kompatible organische antimikrobiell wirksame Verbindung als separate Kitkomponenten vorliegen.

14. Testkit nach Anspruch 12,
    **dadurch gekennzeichnet,**
    daß das Ca-aktivierbare Photoprotein zusammen mit der damit kompatiblen organischen antimikrobiell wirksamen Verbindung vorliegt.

15. Testkit nach einem der Ansprüche 11 bis 14,
    **dadurch gekennzeichnet,**
    daß auch Pufferlösungen oder/und weitere Reagenzlösungen eine mit Ca-aktivierbaren Photoproteinen kompatible organische antimikrobiell wirksame Verbindung enthalten.

16. Verfahren zum Nachweis eines Analyten durch einen Test, bei dem ein Ca-aktivierbares Photoprotein als Komponente verwendet wird,
    **dadurch gekennzeichnet,**
    daß man den Test in Gegenwart eines Stabilisierungsreagenz für das Ca-aktivierbare Photoprotein durchführt, umfassend eine oder mehrere organische antimikrobiell wirksame Verbindungen, worin nach 30 min Testdauer eine restliche Photoproteinaktivität von mindestens 50 % der ursprünglichen Photoproteinaktivität des Ca-aktivierbaren Photoproteins vorhanden ist.

**Europäisches Patentamt**

# EUROPÄISCHER RECHERCHENBERICHT

Nummer der Anmeldung

EP 98 10 8273

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | CHEMICAL ABSTRACTS, vol. 126, no. 1, 7. Juli 1997 Columbus, Ohio, US; abstract no. 4135, XP002074019 * Zusammenfassung * & A.A. SZALY ET AL.: "Bioluminescence and chemiluminescence. Proceedings of the 7th international symposium." 1993 , WILEY , CHICHESTER UK *Zeiten 60-63: K. Flanagan et al.; A study of the stability of Aqualite (recombinant aequorin) lyophilized and in solution in various buffers* --- | 1-16 | G01N33/533 G01N33/53 G01N33/58 |
| A,D | US 5 486 455 A (N.L. STULTS) 23. Januar 1996 * das ganze Dokument * ----- | 1-16 | |

RECHERCHIERTE SACHGEBIETE (Int.Cl.6)

G01N

Der vorliegende Recherchenbericht wurde für alle Patentansprüche erstellt

| Recherchenort | Abschlußdatum der Recherche | Prüfer |
|---|---|---|
| DEN HAAG | 10. August 1998 | Van Bohemen, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)